# EUROPÄISCHE PATENTSCHRIFT

(11) **EP 0 580 641 B1**
(45) Veröffentlichungstag und Bekanntmachung des Hinweises auf die Patenterteilung: **27.12.1996**
(21) Anmeldenummer: 92907730.3
(22) Anmeldetag: 08.04.1992
(51) Int. Cl.: C07D 401/04, A61K 31/445

(54) **NEUE THALIDOMIDDERIVATE, EIN VERFAHREN ZU DEREN HERSTELLUNG SOWIE DIE VERWENDUNG DERSELBEN IN ARZNEIMITTELN**
NEW THALIDOMIDE DERIVATIVES, METHOD OF MANUFACTURE AND USE THEREOF IN MEDICAMENTS
NOUVEAUX DERIVES DE LA THALIDOMIDE, PROCEDE DE FABRICATION ET UTILISATION DESDITS DERIVES DANS DES MEDICAMENTS

(30) Priorität: 17.04.1991 DE 4112566
(43) Veröffentlichungstag der Anmeldung: 02.02.1994
(73) Patentinhaber: Grünenthal GmbH, D-52078 Aachen (DE)
(72) Erfinder: EGER, Kurt, Prof. Dr., D-72072 Tübingen (DE); EHNINGER, Gerhard, Prof. Dr., D-72074 Tübingen (DE); STUHLER, Alfred, Dr., D-89143 Blaubeuren (DE)
(86) Internationale Anmeldenummer: EP9200790
(87) Internationale Veröffentlichungsnummer: WO9218496

(56) Entgegenhaltungen:
- DE-A- 1 545 672
- DE-A- 1 545 706
- DE-A- 1 670 391
- GB-A- 768 821
- SCIENTIA PHARMACEUTICA, 49. Jahrgang, Nr. 1, 30. März 1981, H. KOCH: "Die Arenoxid-Hypothese der Thalidomid-Wirkung. Überlegungen zum molekularen Wirkungsmechanismus des "klassischen" Teratogens", Seiten 67-99
- DESIGN OF PRODRUGS, (H. Bundgaard ed.) chapter 1, 3.3 N-Acyloxyalkyl Derivatives (1985)
- THE LANCET, (1988) S. 117
- ARCHIV DER PHARMAZIE, Weinheim (1988) 321, S. 371-373

## Beschreibung

Die Erfindung betrifft neue Thalidomidderivate der allgemeinen Formel I 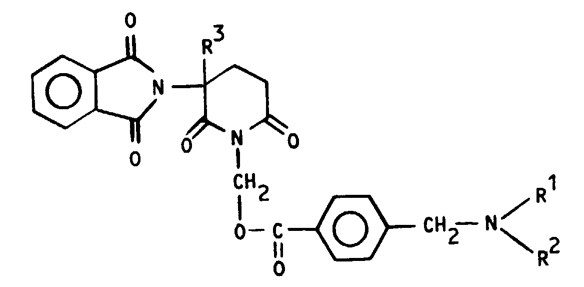 diese Verbindungen enthaltende Arzneimittel sowie ein Verfahren zur Herstellung dieser Verbindungen und Arzneimittel.

Es ist bekannt, daß Thalidomid (3-Phthalimido-piperidin-2,6-dion) immunsuppressiv und immunmodulierend wirksam ist (The Lancet 1988, 117). Für die therapeutische Anwendung dieser Verbindung ist jedoch deren mit 0,012 mg/ml geringe Wasserlöslichkeit [Arch. Pharm. 321, 371 (1988)] von großem Nachteil mit der Folge, daß Thalidomid bisher nur oral verabreicht werden kann und es dadurch bei Erkrankungen, welche mit großen Schleimhautdefekten im Gastrointestinaltrakt einhergehen, wie beispielsweise der Graft-versus-Host-Disease, zu Resorptionsstörungen und damit verbunden zu beträchtlichen Schwankungen der Plasmaspiegel kommt.

Desweiteren sind Thalidomidderivate bekannt, die am Piperidinstickstoff entweder einen substituierten Aminomethylrest (DE 15 45 672, DE 15 45 706, DE 16 70 391) oder einen Kohlenwasserstoffrest (Scientia Pharmaceutica 49, 67 - 99 (1991)) besitzen.

"Design of Prodrugs" edited by Hans Bundgaard, Chapter 1, 3.3 N-Acyloxyalkyl Derivatives (1985) ist zu entnehmen, daß die Einführung einer Acyloxyalkylgruppe in ein schlecht wasserlösliches Arzneimittel sowohl eine Verbesserung als auch eine Verschlechterung der Wasserlöslichkeit bewirken kann. Gemäß Tabelle 9 auf Seite 22 verringert die Einführung der Benzoyloxymethylgruppe die Löslichkeit von Allopurinol um den Faktor 20.

Die der Erfindung zugrundeliegende Aufgabe bestand in der Entwicklung wasserlöslicher Thalidomidderivate, die eine mit Thalidomid mindestens vergleichbare immunsuppressive und immunmodulierende Wirkung besitzen und sich insbesondere zur parenteralen Applikation eignen.

Es wurde gefunden, daß neue Thalidomidderivate die an sie gestellten Anforderungen erfüllen.

Erfindungsgegenstand sind dementsprechend Thalidomidderivate der allgemeinen Formel I 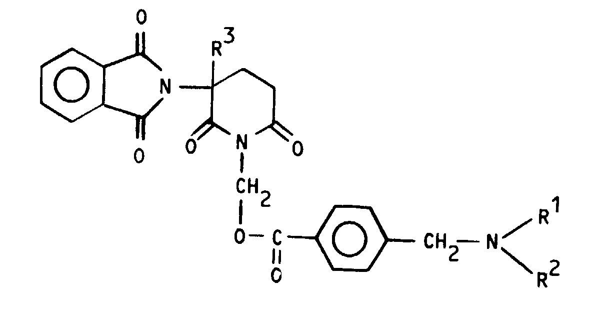 in der die Reste R¹ und R² gleich oder verschieden sind und jeweils eine C₁₋₆-Alkylgruppe oder R¹ und R² zusammen -CH₂CH₂-X-CH₂CH₂- bedeuten, R³ H oder CH₃ und X O oder NH ist, und/oder deren Salze in racemischer oder optisch aktiver Form.

Bevorzugt werden Thalidomidderivate, in denen der Rest R³ H bedeutet. Thalidomidderivate, in denen die Reste R¹ und R² zusammen -CH₂CH₂OCH₂CH₂- oder -CH₂CH₂NHCH₂CH₂-, insbesondere -CH₂CH₂OCH₂CH₂- bedeuten, sind besonders bevorzugt.

Die erfindungsgemäßen Verbindungen sind wasserlöslich und in wäßrig gelöster oder dispergierter Form stabil, so daß sie insbesondere zur parenteralen Applikation an Menschen geeignet sind.

Weiterer Erfindungsgegenstand sind daher Arzneimittel, die als Wirkstoff wenigstens ein Thalidomidderivat und/oder wenigstens ein entsprechendes Salz in racemischer oder optisch aktiver Form erhalten.

Die insbesondere für die parenterale Applikation geeigneten pharmazeutischen Zubereitungsformen, wie Lösungen, Suspensionen, leicht rekonstituierbare Trockenzubereitungen, Salben, Pasten, Gele, Wirkstoffe in einem Depot in gelöster Form oder Pflaster, sind an sich bekannt, und die Einarbeitung der erfindungsgemäßen Verbindungen in diese Zubereitungsformen wirft für den Fachmann keinerlei Probleme auf. Bei der erfindungsgemäßen Herstellung dieser Arzneimittel muß selbstverständlich mit der üblichen Sorgfalt bei Auswahl der Trägermaterialien, Hilfs- und Zusatzstoffe, beispielsweise Lösungsmittel, Verdünnungsmittel, Stabilisatoren, Dispergiermittel, Netzmittel, Bindemittel, Farbstoffe und Aromastoffe, vorgegangen werden. Insbesondere ist auf Sterilität und - sofern die erfindungsgemäßen Arzneimittel in flüssiger Form vorliegen - Isotonie zu achten.

Zur Behandlung von Erkrankungen des Immunsystems wie Lepra, Becet-Syndrom, Lupus erythematodes, Prurigo nodularis, Mundaphten bei Aids-Kranken, Colitis ulcerosa und insbesondere zur Behandlung der Graft-versus-Host-Disease, können die erfindungsgemäßen Arzneimittel intravenös, intradermal, intramuskulär und intranasal sowie örtlich, zum Beispiel auf Infektionen an der Haut, der Schleimhäute und an den Augen, appliziert werden.

Weiterer Erfindunggegenstand ist ein Verfahren zur Herstellung von Thalidomidderivaten der allgemeinen Formel I 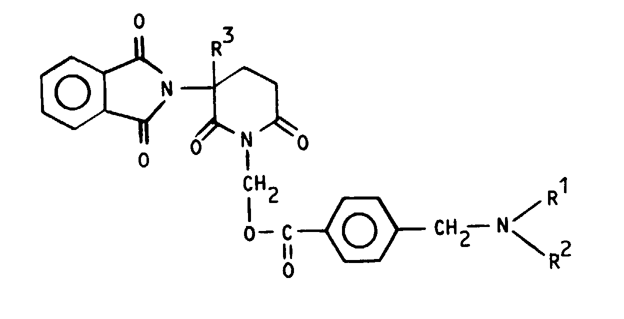 in der die Reste R¹ und R² gleich oder verschieden sind und jeweils eine C₁₋₆-Alkylgruppe oder R¹ und R² zusammen -CH₂CH₂-X-CH₂CH₂- bedeuten, R³ H oder CH₃ und X O oder NH ist, und/oder deren Salze in racemischer oder optisch aktiver Form, welches dadurch gekennzeichnet ist, daß man eine Verbindung der allgemeinen Formel II 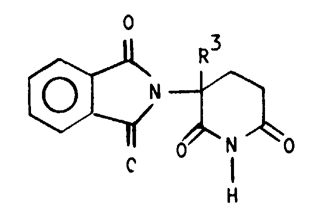 in racemischer oder optisch aktiver Form mit Formaldehyd zu einer N-Hydroxymethylverbindung der allgemeinen Formel III 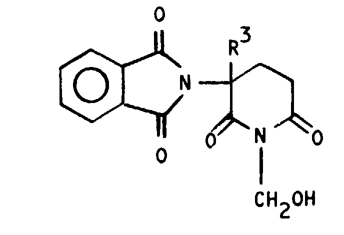 umsetzt, die erhaltene N-Hydroxymethylverbindung mit 4-Chlormethylbenzoesäure in Gegenwart von Dicyclohexylcarbodiimid in einen Ester der allgemeinen Formel IV 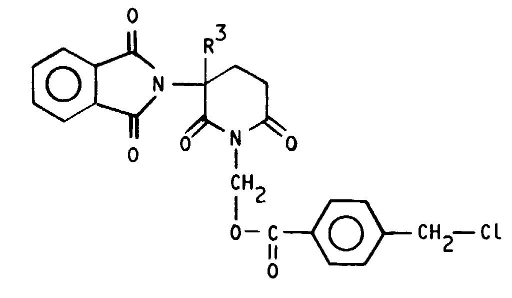 überführt und aus diesem durch Umsetzung mit einem Di(C₁₋₆-alkyl)amin, Morpholin oder Piperazin ein Thalidomidderivat der allgemeinen Formel I herstellt, aus welchem man gegebenenfalls mit einer Säure ein entsprechendes Salz erhält.

Als Ausgangsverbindung zur Herstellung der erfindungsgemäßen Thalidomidderivate wird vorzugsweise Thalidomid eingesetzt, das am Glutarimidstickstoffatom mit Formaldehyd in bekannter Weise hydroxymethyliert wird. Nach Umsetzung mit 4-Chlormethylbenzoesäure in Gegenwart von Dicyclohexylcarbodiimid in einem Lösungsmittel oder Lösungsmittelgemisch wird durch Umsetzung von vorzugsweise Morpholin oder Piperazin, besonders bevorzugt Morpholin, in Gegenwart eines Alkalihalogenids, beispielsweise Natriumjodid, in einem wasserfreien Lösungsmittel oder Lösungsmittelgemisch ein erfindungsgemäßes Thalidomidderivat erhalten, das anschließend gewünschtenfalls mit einer Säure, beispielsweise Salzsäure, gegebenenfalls in Gegenwart eines C₁₋₃-Alkylalkohols in ein entsprechendes Salz überführt wird.

### Beispiel

### Herstellung von N-(4-Morpholinomethyl-benzoyloxymethyl)thalidomid, Hydrochloridsalz

2,58 g Thalidomid wurden in 30 ml 35 gew.%-iger Formaldehyd-Lösung bis zur Bildung einer klaren Lösung unter Rückfluß erhitzt und anschließend auf 20° C abgekühlt. Nach 24 Stunden wurde filtriert, der Rückstand mit 3 gew.%-iger Formaldehydlösung gewaschen und getrocknet. Es wurde N-Hydroxymethyl-thalidomid mit einem Schmelzpunkt von 165° C in 70 %-iger Ausbeute erhalten.

1,1 g N-Hydroxymethyl-thalidomid, 0,68 g 4-Chlormethylbenzoesäure, 1,03 g Dicyclohexylcarbodiimid und 0,06 g 4-Pyrrolidinopyridin in 25 ml Dichlormethan wurden 24 Stunden bei 20° C gerührt. Anschließend wurde Cyclohexylharnstoff abfiltriert und Dichlormethan destillativ entfernt. Der erhaltene Rückstand wurde in Ethanol umkristallisiert. Es wurde N-(4-Chlormethyl-benzoyloxymethyl)thalidomid mit einem Schmelzpunkt von 215 - 220° C in 60 %-iger Ausbeute erhalten.

880 mg N-(4-Chlormethyl-benzoyloxymethyl)thalidomid, 350 mg Morpholin und 20 mg Natriumjodid wurden in absolutem Aceton 24 Stunden bei 20° C gerührt. Anschließend wurde Aceton destillativ entfernt und der ölige Rückstand säulenchromatographisch an Kieselgel 60 (Elutionsmittel: Ethylacetat) gereinigt. Das erhaltene Öl wurde in ethanolischer Salzsäure gelöst und nach Zugabe von Diethylether ausgefällt. Nach Umkristallisieren in Ethanol wurde N-(4-Morpholinomethyl-benzoyloxymethyl)thalidomid, Hydrochloridsalz mit einem Schmelzpunkt von 236° C in 40 %-iger Ausbeute erhalten.

¹H-NMR (200 MHz, DMSO-d₆):
2,08, 2,62 (m, m, 2H, CH₂-CH);
2,84, 2,91 (m, m, 2H, CH₂CO);
3,10, 3,70 (m, m, 8H, -CH₂-CH₂);
4,41 (s, 2H, -CH₂-N);
5,35, 5,43 (dd, 1H, CH-CH₂);
5,92 (d, 2H, N-CH₂-O);
7,72, 7,92 (m, m, 8H, aromat.);
11,20 (s, 1H, H⁺)

## Patentansprüche

1. Thalidomidderivate der allgemeinen Formel I 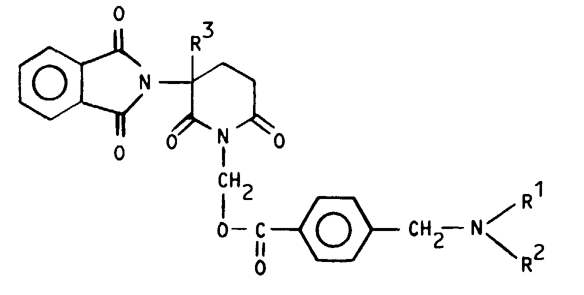 in der die Reste R¹ und R² gleich oder verschieden sind und jeweils eine C₁₋₆-Alkylgruppe oder R¹ und R² zusammen -CH₂CH₂-X-CH₂CH₂- bedeuten, R³ H oder CH₃ und X O oder NH ist, und/oder deren Salze in racemischer oder optisch aktiver Form.

2. Thalidomidderivate nach Anspruch 1, dadurch gekennzeichnet, daß R³ H ist.

3. Thalidomidderivate nach einem oder beiden der Ansprüche 1 bis 2, dadurch gekennzeichnet, daß die Reste R¹ und R² zusammen -CH₂CH₂-X-CH₂CH₂- bedeuten.

4. Thalidomidderivate nach einem oder mehreren der Ansprüche 1 bis 3, dadurch gekennzeichnet, daß die Reste R¹ und R² zusammen -CH₂CH₂-O-CH₂CH₂- bedeuten.

5. Arzneimittel, dadurch gekennzeichnet, daß es als Wirkstoff wenigstens ein Thalidomidderivat und/oder wenigstens ein entsprechendes Salz in racemischer oder optisch aktiver Form gemäß den Ansprüchen 1 bis 4 enthält.

6. Arzneimittel nach Anspruch 5, dadurch gekennzeichnet, daß es zur parenteralen Applikation geeignet ist.

7. Verfahren zur Herstellung eines Arzneimittels gemäß den Ansprüchen 5 bis 6, dadurch gekennzeichnet, daß man wenigstens ein Thalidomidderivat und/oder wenigstens ein entsprechendes Salz in racemischer oder optisch aktiver Form gemäß den Ansprüchen 1 bis 4 in bekannter Weise mit Hilfs- und Zusatzstoffen und gegebenenfalls Trägermaterialien verarbeitet und aus dem erhaltenen Gemisch eine Einzeldosierungsform herstellt.

8. Verfahren zur Herstellung von Thalidomidderivaten der allgemeinen Formel I 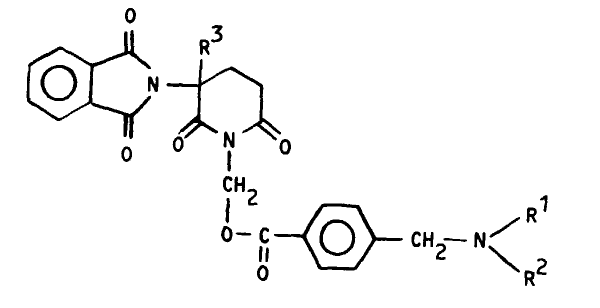 in der die Reste R¹ und R² gleich oder verschieden sind und jeweils eine C₁₋₆-Alkylgruppe oder R¹ und R² zusammen -CH₂CH₂-X-CH₂CH₂- bedeuten, R³ H oder CH₃ und X O oder NH ist, und/oder deren Salze in racemischer oder optisch aktiver Form, dadurch gekennzeichnet, daß man eine Verbindung der allgemeinen Formel II 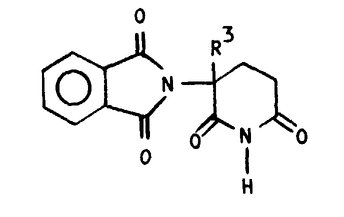 in racemischer oder optisch aktiver Form mit Formaldehyd zu einer N-Hydroxymethylverbindung der allgemeinen Formel III 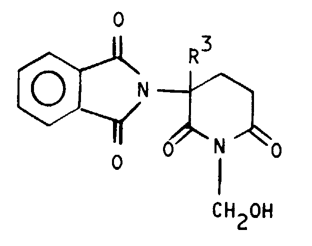 umsetzt, die erhaltene N-Hydroxymethylverbindung mit 4-Chlormethylbenzoesäure in Gegenwart von Dicyclohexylcarbodiimid in einen Ester der allgemeinen Formel IV 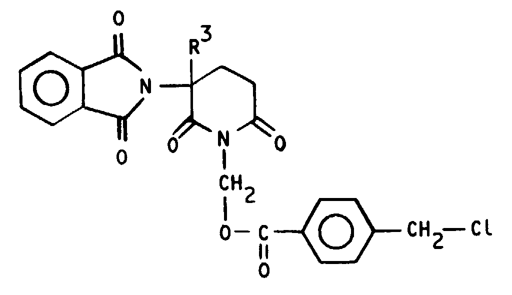 überführt und aus diesem durch Umsetzung mit einem Di(C₁₋₆-alkyl)amin, Morpholin oder Piperazin ein Thalidomidderivat der allgemeinen Formel I herstellt, aus welchem man gegebenenfalls mit einer Säure ein entsprechendes Salz erhält.

9. Verfahren nach Anspruch 8, dadurch gekennzeichnet, daß man einen Ester der allgemeinen Formel IV mit Morpholin oder Piperazin umsetzt.

10. Verfahren nach einem oder beiden der Ansprüche 8 bis 9, dadurch gekennzeichnet, daß man einen Ester der allgemeinen Formel IV mit Morpholin umsetzt.

## Claims

1. Thalidomide derivatives of general formula I: 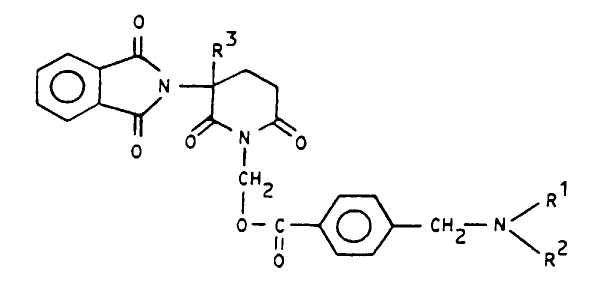 in which the radicals R¹ and R² are identical or different and are each a C₁₋₆-alkyl group, or R¹ and R² together are -CH₂CH₂-X-CH₂CH₂-, R³ is H or CH₃ and X is O or NH, and/or their salts in racemic or optically active form.

2. Thalidomide derivatives according to Claim 1, characterized in that R³ is H.

3. Thalidomide derivatives according to one or both of Claims 1 and 2, characterized in that the radicals R¹ and R² together are -CH₂CH₂-X-CH₂CH₂-.

4. Thalidomide derivatives according to one or more of Claims 1 to 3, characterized in that the radicals R¹ and R² together are -CH₂CH₂-O-CH₂CH₂-.

5. A drug, characterized in that it contains, as active ingredient, at least one thalidomide derivative and/or at least one corresponding salt in racemic or optically active form according to Claims 1 to 4.

6. A drug according to Claim 5, characterized in that it is suitable for parenteral administration.

7. A process for the preparation of a drug according to Claims 5 and 6, characterized in that at least one thalidomide derivative and/or at least one corresponding salt in racemic or optically active form according to Claims 1 to 4 are processed in known manner with auxiliary substances and additives and optionally excipients, and a single-dosage form is prepared from the resulting mixture.

8. A process for the preparation of thalidomide derivatives of general formula I: 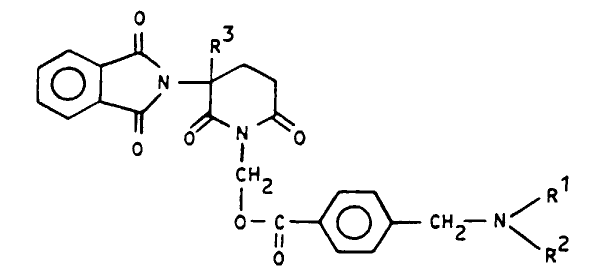 in which the radicals R¹ and R² are identical or different and are each a C₁₋₆-alkyl group, or R¹ and R² together are -CH₂CH₂-X-CH₂CH₂-, R³ is H or CH₃ and X is O or NH, and/or their salts in racemic or optically active form, characterized in that a compound of general formula II: 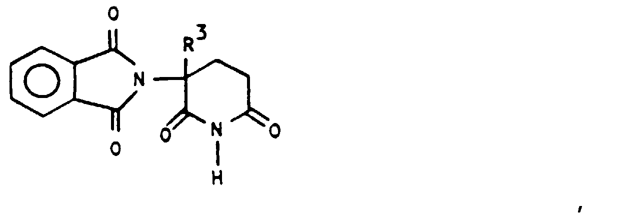 in racemic or optically active form, is reacted with formaldehyde to give an N-hydroxymethyl compound of general formula III: 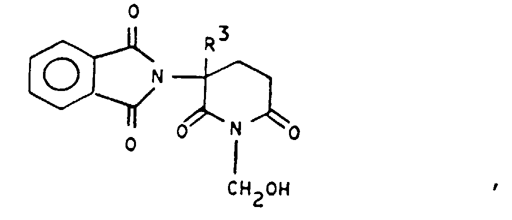 the resulting N-hydroxymethyl compound is reacted with 4- chloromethylbenzoic acid in the presence of dicyclohexylcarbodiimide to give an ester of general formula IV: 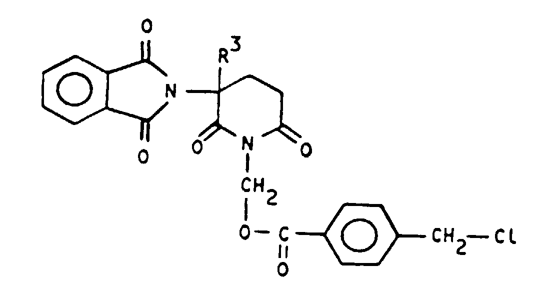 and this is reacted with a di(C₁₋₆-alkyl)amine, morpholine or piperazine in order to prepare a thalidomide derivative of general formula I, from which a corresponding salt is optionally obtained with an acid.

9. A process according to Claim 8, characterized in that an ester of general formula IV is reacted with morpholine or piperazine.

10. A process according to one or both of Claims 8 and 9, characterized in that an ester of general formula IV is reacted with morpholine.

## Revendications

1. Dérivés de thalidomide de formule générale I 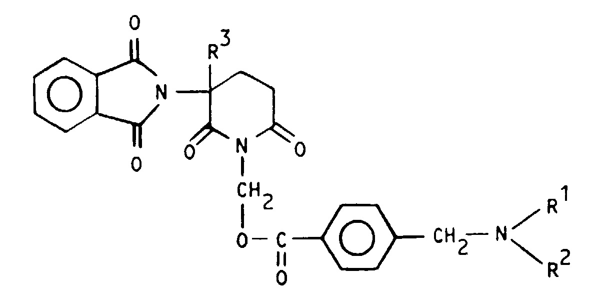 dans laquelle les restes R¹ et R² sont identiques ou différents et représentent chacun un groupe alkyle en C₁ à C₆, ou bien R¹ et R² forment ensemble un groupe -CH₂CH₂-X-CH₂CH₂-, R³ est de l'hydrogène ou un groupe CH₃ et X est de l'oxygène ou un groupe NH, et/ou leurs sels sous la forme racémique ou optiquement active.

2. Dérivés de thalidomide suivant la revendication 1, caractérisés en ce que R³ est de l'hydrogène.

3. Dérivés de thalidomide suivant l'une ou l'autre des revendications 1 et 2 ou les deux, caractérisés en ce que les restes R¹ et R² forment ensemble un groupe -CH₂CH₂-X-CH₂CH₂-.

4. Dérivés de thalidomide suivant une ou plusieurs des revendications 1 à 3, caractérisés en ce que les restes R¹ et R² forment ensemble un groupe -CH₂CH₂-O-CH₂CH₂-.

5. Médicament, caractérisé en ce qu'il contient comme substance active au moins un dérivé de thalidomide et/ou au moins un sel correspondant sous la forme racémique ou optiquement active selon les revendications 1 à 4.

6. Médicament suivant la revendication 5, caractérisé en ce qu'il convient pour l'administration parentérale.

7. Procédé de préparation d'un médicament suivant les revendications 5 et 6, caractérisé en ce qu'on met en oeuvre au moins un dérivé de thalidomide et/ou au moins un sel correspondant sous la forme racémique ou optiquement active suivant les revendications 1 à 4 d'une manière connue avec des substances auxiliaires et des additifs et le cas échéant des supports et on prépare une forme dosée individuelle à partir du mélange obtenu.

8. Procédé de production de dérivés de thalidomide de formule générale I 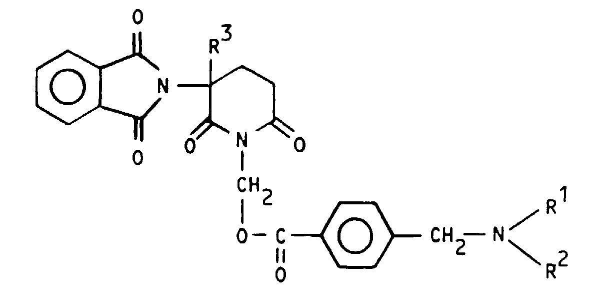 dans laquelle les restes R¹ et R² sont identiques ou différents et représentent chacun un groupe alkyle en C₁ à C₆, ou bien R¹ et R² forment ensemble un groupe -CH₂CH₂-X-CH₂CH₂-, R³ est de l'hydrogène ou un groupe CH₃ et X est de l'oxygène ou un groupe NH, et/ou de leurs sels sous la forme racémique ou optiquement active, caractérisé en ce qu'on fait réagir un composé de formule générale II 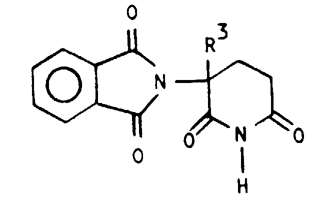 sous la forme racémique ou optiquement active avec du formaldéhyde pour former un composé N-hydroxyméthylé de formule générale III 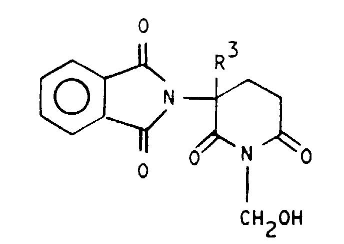 on transforme le composé N-hydroxyméthylé obtenu, avec l'acide 4-chlorométhylbenzoïque en présence de dicyclohexylcarbodiimide, en un ester de formule générale IV 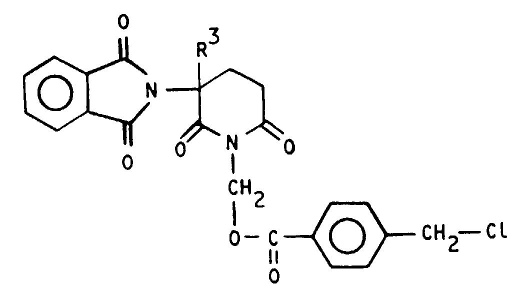 et on prépare à partir de cet ester, par réaction avec une di(alkyle en C₁ à C₆)amine, la morpholine ou la pipérazine, un dérivé de thalidomide de formule générale I à partir duquel on obtient éventuellement un sel correspondant avec un acide.

9. Procédé suivant la revendication 8, caractérisé en ce qu'on fait réagir un ester de formule générale IV avec la morpholine ou la pipérazine.

10. Procédé suivant l'une des revendications 8 et 9 ou les deux, caractérisé en ce qu'on fait réagir un ester de formule générale IV avec la morpholine.
